# EUROPEAN PATENT APPLICATION

(11) **EP 2 912 989 A1**
(43) Date of publication of application: **02.09.2015**
(21) Application number: 14827108.3
(22) Date of filing: 13.05.2014
(51) Int. Cl.: A61B 1/00, A61B 1/12, G02B 23/24

(54) **ENDOSCOPE TRANSPORT APPARATUS**

(30) Priority: 17.07.2013 JP 2013148642
(71) Applicant: OLYMPUS CORPORATION, Shibuya-ku Tokyo 151-0072 (JP)
(72) Inventor: SUZUKI Eiri, Tokyo 151-0072 (JP)
(74) Representative: Gunzelmann, Rainer
(86) International application number: PCT/JP2014/062742
(87) International publication number: WO 2015/008527

(57) **Abstract**

An endoscope transportation instrument of the invention includes: a holding portion that holds an endoscope and includes a through hole for discharging liquid dripping from the endoscope when the endoscope is arranged at an upper position; and a liquid receiving portion arranged so as to intersect with the holding portion, the liquid receiving portion including a liquid-tight recessed portion that opens upward and receives the liquid dripping from the endoscope when the endoscope held by the holding portion is arranged at the upper position.

## Description

### Technical Field

The present invention relates to an endoscope transportation instrument which is able to hold an endoscope.

### Background Art

Endoscopes for use in medical fields are subjected to cleaning processing and disinfecting processing after use by using a cleaning/disinfecting apparatus, as disclosed in Japanese Patent Application Laid-Open Publication No. 2009-172055, for example. Before automated processing to be performed on the endoscope after use by using the cleaning/disinfecting apparatus, preliminary cleaning is performed for removing dirt adhered to the endoscope with running water or by using a brush in a sink.

When the endoscope subjected to the preliminary cleaning is transported from the sink to the cleaning/disinfecting apparatus, the endoscope has not been subjected to disinfecting processing yet and is wet. Therefore, it is necessary to avoid adherence of liquid droplets dripping from the endoscope to the floor or other members.

The present invention has been achieved in view of the above-described points, and an object of the present invention is to provide an endoscope transportation instrument that can transport an endoscope, while preventing dripping of liquid droplets from the endoscope.

### Disclosure of Invention

### Means for Solving the Problem

An endoscope transportation instrument according to one aspect of the present invention is an endoscope transportation instrument for transporting an endoscope in a wet state, and the endoscope transportation instrument includes: a holding portion that holds the endoscope and includes a through hole for discharging liquid dripping from the endoscope when the endoscope is arranged at an upper position; and a liquid receiving portion arranged so as to intersect with the holding portion, the liquid receiving portion including a liquid-tight recessed portion that opens upward and receives the liquid dripping from the endoscope when the endoscope held by the holding portion is arranged at the upper position.

### Brief Description of the Drawings

FIG. 1 is a front view of an endoscope transportation instrument according to a first embodiment in a state where the endoscope transportation instrument holds an endoscope.
FIG. 2 is a perspective view of the endoscope transportation instrument according to the first embodiment in the state where the endoscope transportation instrument holds the endoscope.
FIG. 3 is a front view of the endoscope transportation instrument according to the first embodiment.
FIG. 4 is a perspective view of the endoscope transportation instrument according to the first embodiment.
FIG. 5 is a side view of the endoscope transportation instrument according to the first embodiment.
FIG. 6 is a perspective view showing a configuration of a winding portion.
FIG. 7 illustrates a modified example of a support face portion.
FIG. 8 illustrates a state where the endoscope transportation instrument according to the first embodiment is placed in a processing tank, when viewed from above.
FIG. 9 describes a procedure for housing the endoscope into the endoscope transportation instrument according to the first embodiment.
FIG. 10 describes a procedure for housing the endoscope into the endoscope transportation instrument according to the first embodiment.
FIG. 11 describes a procedure for housing the endoscope into the endoscope transportation instrument according to the first embodiment.
FIG. 12 illustrates a state where the endoscope transportation instrument according to the first embodiment is used above the sink.
FIG. 13 illustrates a modified example of the winding portion.
FIG. 14 is a front view of an endoscope transportation instrument according to a second embodiment.
FIG. 15 is a side view of the endoscope transportation instrument according to the second embodiment.
FIG. 16 illustrates a modified example of the endoscope transportation instrument according to the second embodiment.

### Best Mode for Carrying Out the Invention

Hereinafter, preferred embodiments of the present invention will be described with reference to drawings. Note that, in each of the drawings used for the description below, a different scale size is used for each of the constituent elements in order to allow each of the constituent elements to be illustrated in a recognizable size in the drawings, and the present invention is not limited to the number, shapes, ratio of the sizes of the constituent elements, and a relative positional relationship among the constituent elements shown in these drawings.

### (First Embodiment)

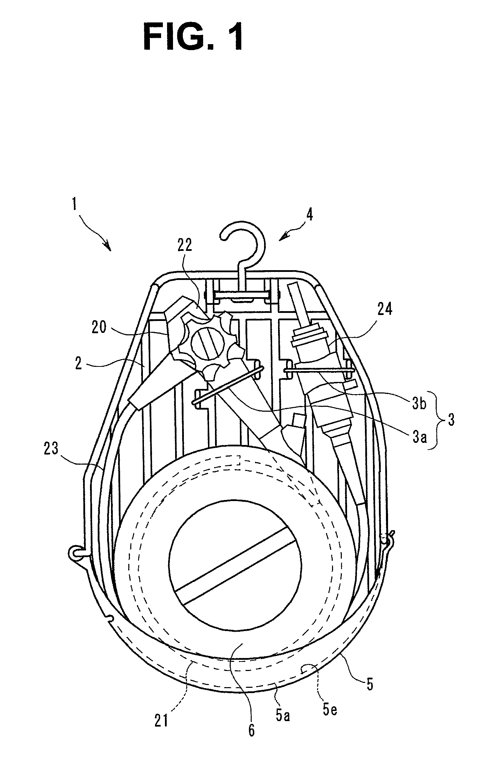

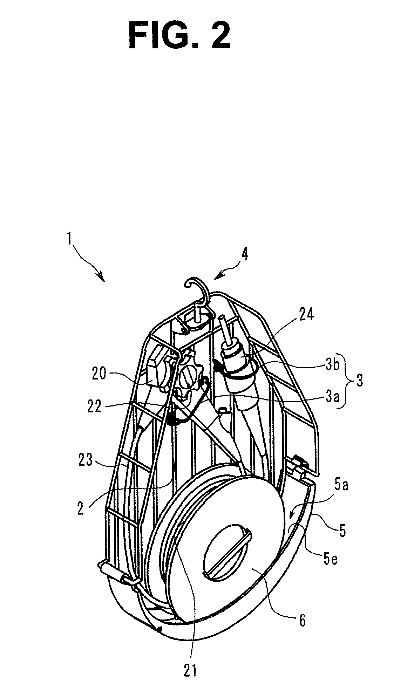

Below, description will be made on an example of the first embodiment of the present invention. As shown in FIGS. 1 and 2, an endoscope transportation instrument 1 of the present embodiment is an instrument configured to transport an endoscope 20 including an insertion portion 21 and hold the insertion portion 21.

Since the endoscope 20 is a well-known one, the configuration thereof is not particularly limited, and detailed description thereof will be omitted. Roughly, the endoscope 20 includes an elongated insertion portion 21 to be introduced into a subject, an operation portion 22 provided on the proximal end side of the insertion portion 21, a universal cord 23 extended from the operation portion 22, and a connector portion 24 provided to the universal cord 23. The insertion portion 21 has flexibility except for the vicinity of the distal end portion.

The universal cord 23 and the connector portion 24 are configured to connect the endoscope 20 to external apparatuses such as a video processor, a suction device, and the like. Therefore, if the endoscope 20 is configured to be operable independently without a need to be connected to the external apparatuses, the universal cord 23 and the connector portion 24 are not required. That is, the endoscope 20 held by the endoscope transportation instrument 1 of the present embodiment has only to be the one configured by including at least the insertion portion 21 and the operation portion 22.

Note that the subject into which the insertion portion 21 of the endoscope 20 is inserted is not limited to a human body and may be another living body, or may be a structure such as a machine or a building. In addition, the configuration of the endoscope 20 is not limited to the one for picking up only an optical image in the subject, and may be the one for picking up a tomographic image of the subject.

The endoscope transportation instrument 1 includes a liquid receiving portion 5 and a holding portion 2.

The liquid receiving portion 5 is a plate-shaped member including a recessed portion 5a. The liquid receiving portion 5 has a liquid-tight configuration which enables liquid to be received and retained in the recessed portion 5a when the recessed portion 5a takes a posture to open in a substantially upward direction. That is, the recessed portion 5a does not include, on a bottom surface portion 5e thereof, a through hole for allowing liquid to pass through.

The holding portion 2 is configured to support the endoscope 20 above the recessed portion 5a when the recessed portion 5a of the liquid receiving portion 5 takes the posture to open upward. In other words, the liquid receiving portion 5 is disposed such that the recessed portion 5a opens toward a position at which the endoscope 20 is supported by the holding portion 2. In addition, the holding portion 2 holds the endoscope 20 such that atmosphere around the endoscope transportation instrument 1 touches the outer surface of the endoscope 20.

Specifically, the holding portion 2 includes a support face portion 2a, an endoscope latching portion 3, and a winding portion 6. The support face portion 2a is a planar member and includes a plurality of through holes 2b. The support face portion 2a may have a shape along a plane or a shape along a curved surface such as a cylindrical surface, a spherical surface, or the like. Furthermore, the support face portion 2a may be provided with a flexing portion. The through holes 2b are configured to remove (discharge) liquid dripping from the endoscope 20 from the support face portion 2a, when the support face portion 2a takes a posture such that the surface thereof is substantially horizontal and the endoscope 20 is disposed at the upper position of the support face portion 2a. The support face portion 2a of the holding portion 2 is arranged so as to intersect with the bottom surface portion 5e of the recessed portion 5a of the liquid receiving portion 5, which enables the endoscope 50 held by the holding portion 2 to be arranged over the recessed portion 5a of the liquid receiving portion 5. In addition, the holding portion 2 is provided with a hook 4 to be described later.

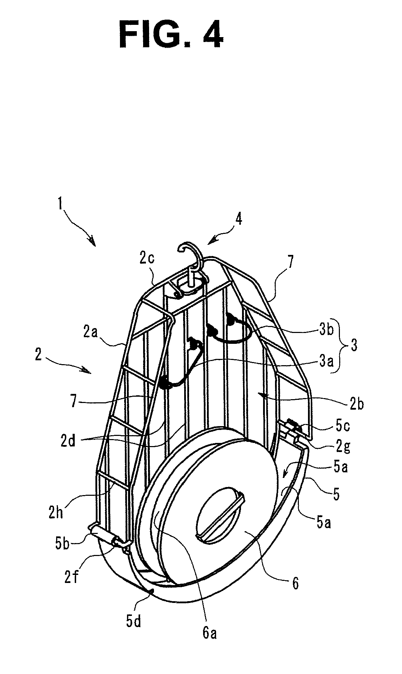

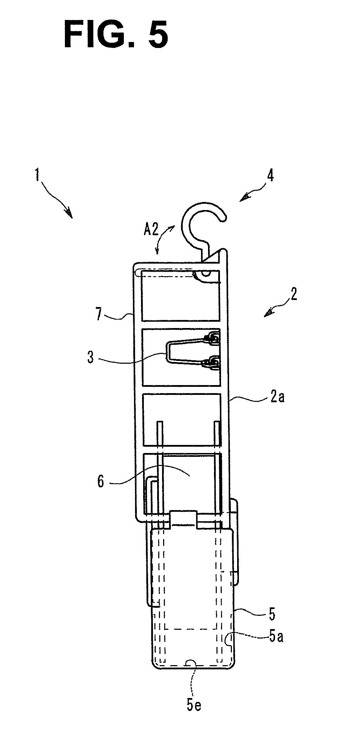

In the present embodiment, as one example, the support face portion 2a is configured by a grating member along a predetermined plane as shown in FIGS. 4 and 5. The support face portion 2a includes a frame member 2c disposed on the outer periphery of the support face portion 2a. The member constituting the grating is disposed in an area surrounded by the frame member 2c. In the present embodiment, clearances between rod-shaped members 2d which constitute the grating are the through holes 2b.

Note that the shape of the grating of the support face portion 2a and a ratio of the area of the through holes 2b to the area of the support face portion 2a are not particularly limited. For example, the grating may be formed by arranging the rod-shaped members so as to intersect with each other. If the frame member 2c has a sufficient strength, the grating disposed inside the frame member may be a net made of string members.

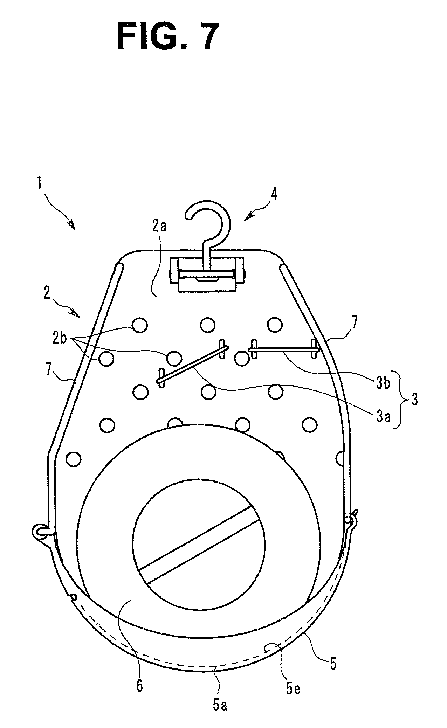

Furthermore, as shown in FIG. 7 as a modified example, the support face portion 2a may be configured by a flat plate member including a plurality of through holes 2b. In the modified example shown in FIG. 7, the support face portion 2a is made of a flat plate member, which enables the support face portion 2a to have increased rigidity.

On one surface of the support face portion 2a which is a planar member, the endoscope latching portion 3 and the winding portion 6 are disposed. The endoscope latching portion 3 is a member for latching at least the operation portion 22 of the endoscope 20 onto the support face portion 2a. The winding portion 6 is a member for latching the insertion portion 21 of the endoscope 20 onto the support face portion 2a, with the insertion portion 21 being in a wound state. The endoscope 20 is supported on the one surface of the support face portion 2a by the endoscope latching portion 3 and the winding portion 6.

In the present embodiment, the endoscope latching portion 3 includes a first supporting portion 3 a that supports the operation portion 22, and a second supporting portion 3b that supports the connector portion 24, as one example. The first supporting portion 3a and the second supporting portion 3b are band-shaped members having flexibility, and both end portions of each of the first supporting portion and second supporting portion are latched onto the support face portion 2a in the state where the first supporting portion and the second supporting portion are wrapped around the operation portion 22 and the connector portion 24, respectively.

The first supporting portion 3a and the second supporting portion 3b have lengths long enough for the first supporting portion and the second supporting portion not to closely contact the outer surfaces of the operation portion 22 and the connector portion 24, respectively, in the state wrapped around the operation 22 and the connector portion 24. In other words, in the state where the endoscope 20 is supported by the holding portion 2, a clearance is created between the endoscope latching portion 3 and the outer surface of the endoscope 20. Note that the configuration of the endoscope latching portion 3 is not limited to the present embodiment, and the endoscope latching portion 3 may be a claw-like rigid member.

In addition, the winding portion 6 is a reel-shaped member around which the insertion portion 21 is wound, as one example in the present embodiment. More specifically, the winding portion 6 is a cylindrical member, and includes a groove portion 6a formed on an outer circumference of the cylindrical surface in the circumferential direction. The insertion portion 21 is wound around the winding portion 6 along the groove portion 6a.

The winding portion 6 is disposed between the endoscope latching portion 3 and the liquid receiving portion 5. In other words, when the recessed portion 5a of the liquid receiving portion 5 is in the posture opening upward, the winding portion 6 is disposed right above the liquid receiving portion 5.

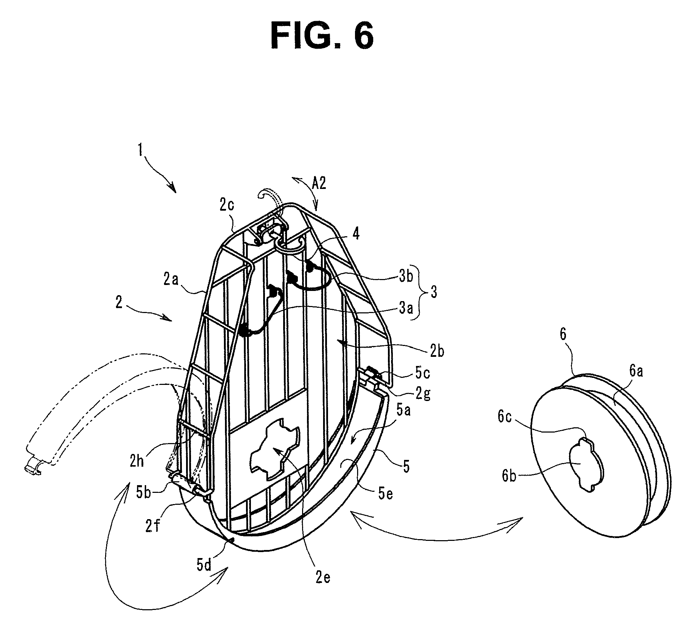

In addition, as shown in FIG. 6, the winding portion 6 is configured to be attachable to and detachable from the support face portion 2a, as one example in the present embodiment. In the present embodiment, as one example of the attaching/detaching portion for allowing the winding portion 6 to be attachable to and detachable from the support face portion 2a, the winding portion 6 is provided with a protruding portion 6b that protrudes one side along the central axis of the winding portion 6. The winding portion 6 is attachably/detachably fixed to the support face portion 2a by fitting the protruding portion 6b into an engaging hole portion 2e provided on the support face portion 2a.

In addition, in the present embodiment, a claw-like portion 6c that protrudes outward in the radial direction is provided at the distal end portion of the protruding portion 6b. The protruding portion 6b can be fixed so as not to fall off from the engaging hole portion 2e by rotationally moving the winding portion 6 around the central axis in the state where the protruding portion 6b is inserted into the engaging hole portion 2e such that the claw-like portion 6c protrudes from the opposite-side surface of the support face portion 2a.

Note that the configuration that allows the winding portion 6 to be attachable to and detachable from the support face portion 2a is not specifically limited and the winding portion 6 may be configured to be fixed to the support face portion 2a with a screw structure, a clamp structure, or the like. Alternatively, the winding portion 6 may be configured to be secured to the support face portion 2a.

In addition, the holding portion 2 of the present embodiment includes a hook 4 and a protection frame 7. The hook 4 is a claw-like member for suspending the endoscope transportation instrument 1 from another rod-like member or a string member.

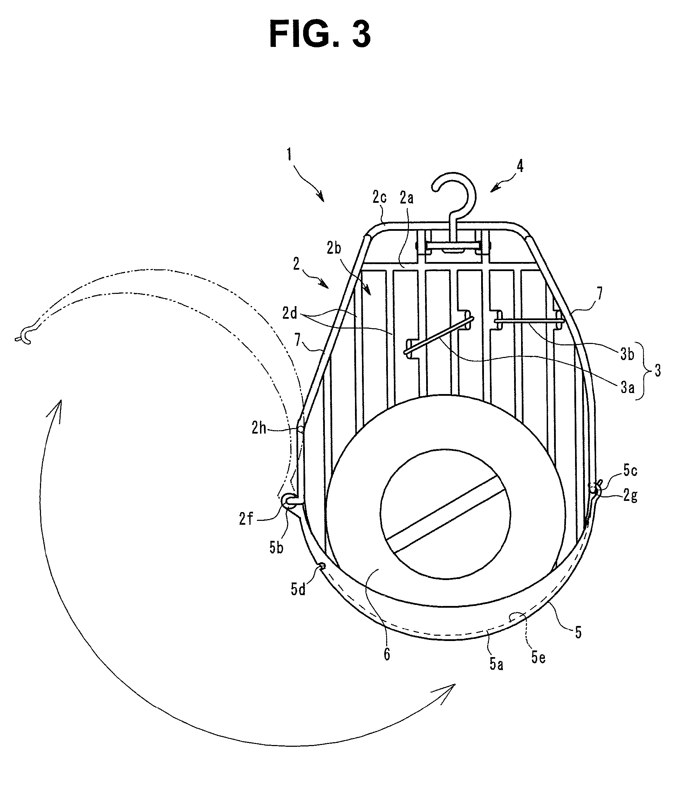

In the state where the endoscope transportation instrument 1 is suspended from another member by the hook 4, when the recessed portion 5a of the liquid receiving portion 5 opens upward (upper side in the drawings), the hook 4 is disposed at a position such that the holding portion 2 is positioned above the recessed portion 5a, as shown in FIGS. 3 and 5, for example. That is, the hook 4 is disposed opposite side of the liquid receiving portion 5 across the holding portion 2.

The base portion of the hook 4 according to the present embodiment is configured to be rotationally movable as shown with the arrow A2 in FIGS. 5 and 6. Specifically, the hook 4 is movable between the two positions, i.e., the using position at which the hook 4 protrudes more outward than the outer edge of the support face portion 2a and the housed position at which the hook 4 is housed within the outer edge of the support face portion 2a, when the support face portion 2a is viewed in planar view (viewpoint in FIG. 3). When the endoscope transportation instrument 1 is suspended by using the hook 4, the hook 4 is positioned at the using position.

The protection frame 7 is one or a plurality of frame-like members vertically provided so as to be substantially perpendicular to the planar support face portion 2a on the one surface of the support face portion 2a, the endoscope latching portion 3 and the winding portion 6 being disposed on the one surface. The protection frame 7 is provided along the outer edge portion of the support face portion 2a, and includes opening portions as through holes for allowing the liquid flowing in the direction along the support face portion 2a to pass through.

The protection frame 7 has a height protruding further than the endoscope 20 latched onto the one surface of the support face portion 2a by the endoscope latching portion 3, to thereby provide an effect for preventing the contact between the endoscope 20 and other members. In the present embodiment, as one example, the protection frame 7 is disposed around the positions at which the operation portion 22 and the connector portion 24 are latched. Note that the protection frame 7 may be provided to entirety of the outer edge portion of the support face portion 2a.

The liquid receiving portion 5 is supported by the holding portion 2. As described above, when the endoscope transportation instrument 1 is suspended by the hook 4, the liquid receiving portion 5 is located below the holding portion 2 and disposed such that the recessed portion 5a opens upward. In addition, in this case, the liquid receiving portion 5 has such a shape that the outer shape of the endoscope 20 supported by the holding portion 2 is fitted within the opening portion of the recessed portion 5a when viewing the endoscope transportation instrument 1 from above.

More specifically, in the present embodiment, when the endoscope transportation instrument 1 is suspended by the hook 4, the support face portion 2a, the endoscope latching portion 3, and the winding portion 6 are disposed above the recessed portion 5a of the liquid receiving portion 5, as shown in FIGS. 3 and 5. In other words, the liquid receiving portion 5 includes the recessed portion 5a having the opening portion which has a shape allowing the outer shapes of the support face portion 2a, endoscope latching portion 3, and the winding portion 6 to be fitted therein when the endoscope transportation instrument 1 suspended by the hook 4 is viewed from above.

The liquid receiving portion 5 is disposed such that the bottom surface portion 5e of the recessed portion 5a which is recessed in an arc shape is close to the outer circumference of the winding portion 6, and a part of the winding portion 6 enters into the recessed portion 5a when the support face portion 2a is viewed in planar view as shown in FIG. 3. The bottom surface portion 5e of the recessed portion 5a is substantially perpendicular to the support face portion 2a and has a shape along the surface bent along the outer circumference of the cylindrical-shaped winding portion 6. In other words, the bottom surface portion 5e of the recessed portion 5a has a substantially cylindrical shape along the bending of the insertion portion 21 on the outside of the insertion portion 21 wound around the winding portion 6 to be bent.

In the present embodiment, the bottom surface portion 5e of the recessed portion 5a is configured by a cylindrical surface having a diameter larger than the diameter of the outer circumference of the winding portion 6, as one example. The bottom surface portion 5e as a cylindrical surface has a central axis which is substantially parallel to the central axis of the winding portion 6.

Note that the bottom surface portion 5e of the recessed portion 5a is not limited to the cylindrical shape as long as the bottom surface 5e has a shape substantially along the outer circumference of the winding portion 6. For example, the bottom surface portion 5e of the recessed portion 5a may be a polyhedron configured by a plurality of planes, or may be an elliptic cylindrical shape.

In addition, in the present embodiment, the value of the depth of the recessed portion 5a is smaller than the value of the radius of the winding portion 6. The liquid receiving portion 5 is made of a member having a predetermined thickness, and has a substantially crescent outer shape curved in an arch shape when the support face portion 2a is viewed in planar view.

The liquid receiving portion 5 may be configured to be fixed to the holding portion 2, or may be configured to be movable or attachable/detachable with respect to the holding portion 2.

In the present embodiment, the liquid receiving portion 5 is movable with respect to the holding portion 2, as one example. As described above, the liquid receiving portion 5 is movable between a first position at which the bottom surface portion 5e of the recessed portion 5a is close to the outer circumference of the winding portion 6 and a second position at which the bottom surface portion 5e of the recessed portion 5a is away from the winding portion 6. In FIGS. 3 and 6, the position of the liquid receiving portion 5 shown by the solid lines is the first position and the position shown by the two-dot chain lines is the second position.

More specifically, the holding portion 2 of the present embodiment is provided with a rotational movement shaft portion 2f which supports the one end of the liquid receiving portion 5, which has the outer surface curved in an arch shape, in a rotationally movable manner. The rotational movement shaft portion 2f is a spindle-like member provided vertically so as to be substantially perpendicular to the planar support face portion 2a on the one surface of the support face portion 2a on which the endoscope latching portion 3 and the winding portion 6 are disposed.

The liquid receiving portion 5 includes a shaft receiving portion 5b connected to the one end of the liquid receiving portion so as to be rotationally movable around the rotational movement shaft portion 2f, and thereby the liquid receiving portion 5 is movable between the first position and the second position. In addition, on the support face portion 2a, a first lock portion 2g that locks the liquid receiving portion 5 at the first position and a second lock portion 2h that locks the liquid receiving portion 5 at the second position are disposed.

The first lock portion 2g is a spindle-like member provided vertically on the one surface of the support face portion 2a so as to be substantially perpendicular to the planar support face portion 2a. The liquid receiving portion 5 is fixed at the first position by a first hook portion 5c provided at the other end of the liquid receiving portion 5 being locked onto the first lock portion 2g.

The second lock portion 2h is a spindle-like member provided vertically on the one surface of the support face portion 2a so as to be substantially perpendicular to the planar support face portion 2a. The liquid receiving portion 5 is fixed at the second position by a second hook portion 5d provided to the liquid receiving portion 5 being locked onto the second lock portion 2h.

In the present embodiment, when the endoscope transportation instrument 1 is suspended by the hook 4 and the liquid receiving portion 5 is fixed at the second position, the liquid receiving portion 5 is away from the position below the holding portion 2.

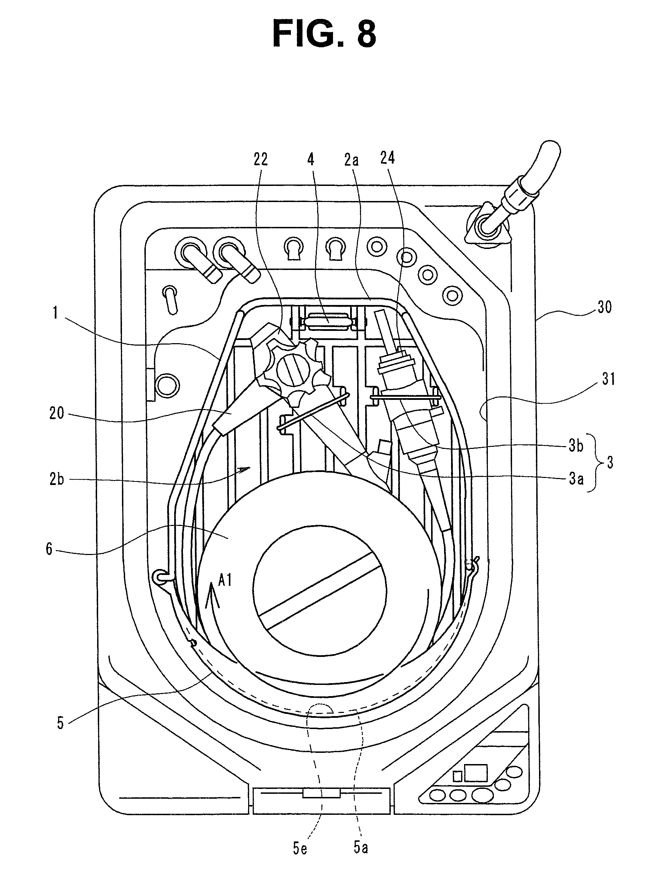

In addition, as shown in FIG. 8, the endoscope transportation instrument 1 according to the present embodiment can be placed in a processing tank 31 of an endoscope cleaning/disinfecting apparatus 30, with the endoscope 20 held in the endoscope transportation instrument 1. In this case, the support face portion 2a of the holding portion 2 is located on the downside or upside in the processing tank 31. In addition, the hook 4 is located at the housed position and the liquid receiving portion 5 is located at the first position. Since the hook 4 can be thus located at the housed position at which the hook 4 is fitted within the outer edge of the support face portion 2a when the support face portion 2a is viewed in planar view, the endoscope transportation instrument 1 can be easily placed in the small-sized processing tank 31.

As described above, the support face portion 2a and the protection frame 7 are provided with the opening portions and the endoscope latching portion 3 can create a clearance between itself and the outer surface of the endoscope 20. Therefore, in the processing tank 31, liquid such as cleaning solution, disinfectant solution, tap water, or the like evenly contacts the endoscope 20 without being blocked by the endoscope transportation instrument 1.

In addition, in the processing tank 31, the bottom surface portion 5e of the recessed portion 5a of the liquid receiving portion 5 is close to the outer circumference of the winding portion 6, which allows the liquid around the winding portion 6 to flow along the bottom surface portion 5e of the recessed portion 5 as shown by the arrow A1 in FIG. 8. That is, presence of the liquid receiving portion 5 allows the liquid in the processing tank 31 to flow along the longitudinal direction of the insertion portion 21 wound around the winding portion 6. The above-described configuration enables the processing using the liquid such as cleaning solution, disinfectant solution, tap water, or the like to be performed sufficiently on the endoscope 20 held by the endoscope transportation instrument 1.

Next, description will be made on the procedure of holding the endoscope 20 by the endoscope transportation instrument 1 having the above-described configuration.

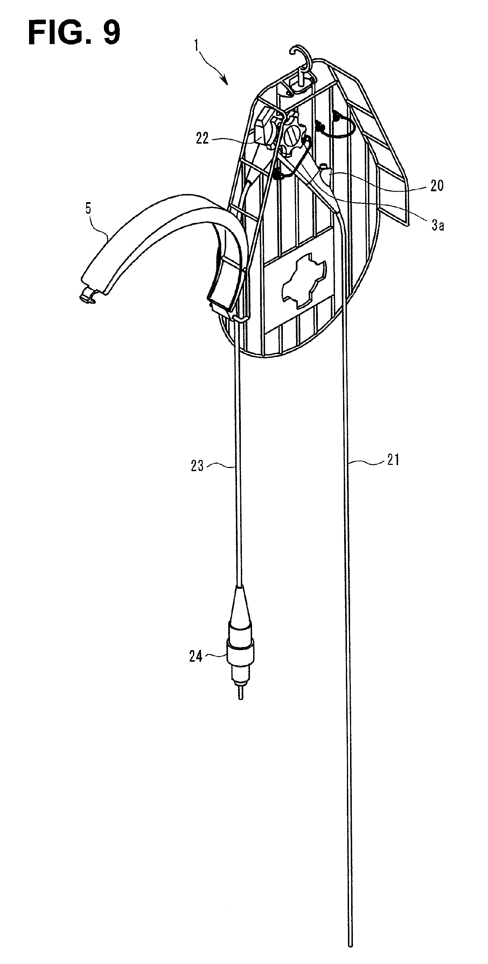

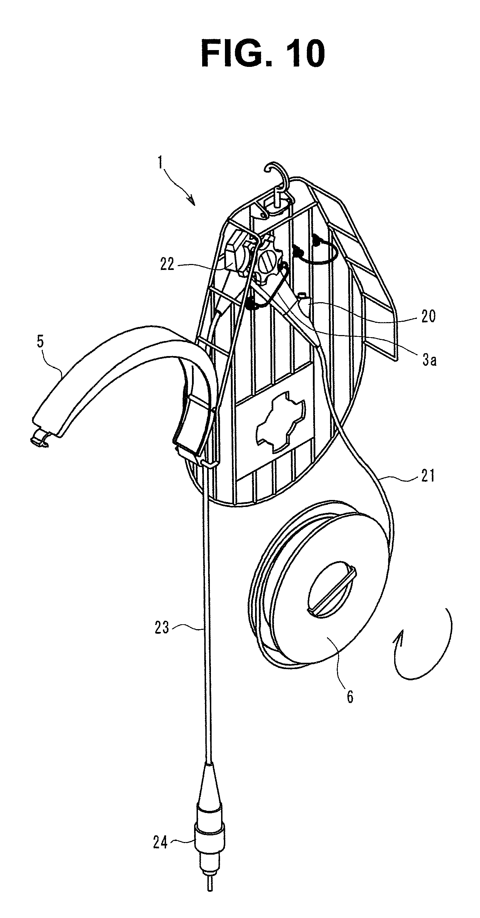

First, as shown in FIG. 9, the operation portion 22 is supported with respect to the support face portion 2a by the first supporting portion 3 a in the state where the liquid receiving portion 5 is fixed at the second position. Next, as shown in FIG. 10, the insertion portion 21 is wound around the winding portion 6 which is in the state detached from the support face portion 2a.

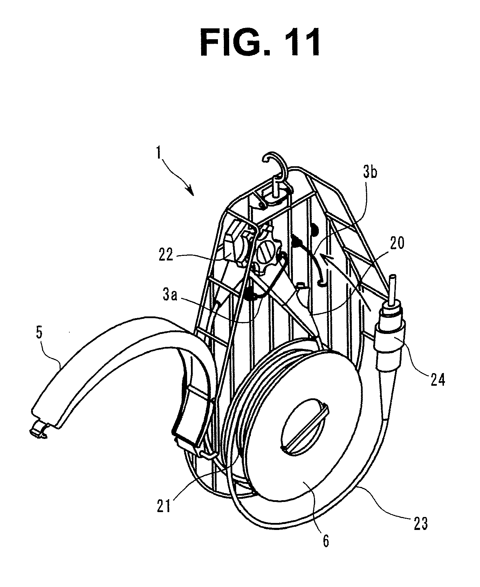

Next, as shown in FIG. 11, the winding portion 6 is fixed to the support face portion 2a, and thereafter the connector portion 24 is supported with respect to the support face portion 2a by the second supporting portion 3b. Note that, in the procedure in FIG. 11, the universal cord 23 is wound around the winding portion 6 as needed. Then, the liquid receiving portion 5 is rotationally moved to the first position and fixed, and thereby bringing the endoscope 20 into the state held by the endoscope transportation instrument 1, as shown in FIG. 2.

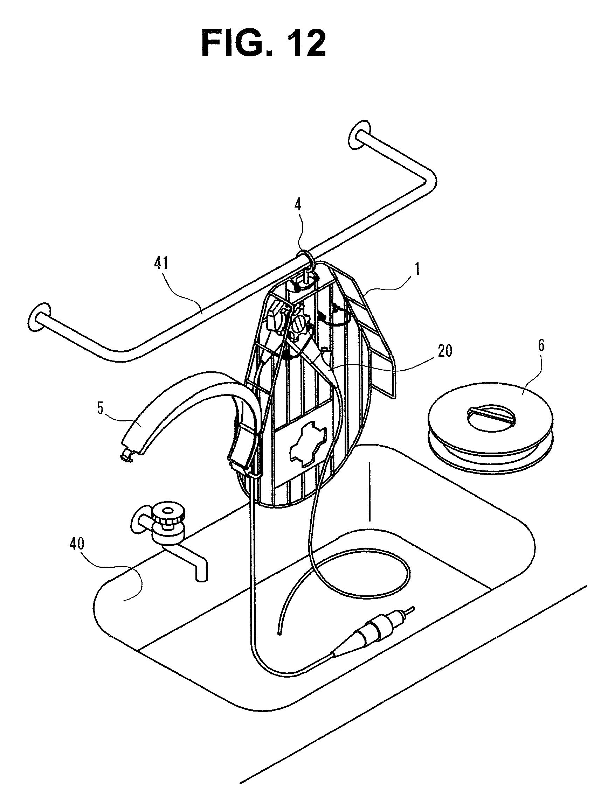

For example, as shown in FIG. 12, if a bridging bar 41 is provided above the sink 40 in which preliminary cleaning of the endoscope 20 is performed, and the hook 4 of the endoscope transportation instrument 1 is locked onto the bridging bar 41, the endoscope 20 subjected to the preliminary cleaning can be brought into the state held by the endoscope transportation instrument 1 above the sink 40 by the above-described procedure. Note that, when the bridging bar 41 is not provided, the operation can be performed by placing the endoscope transportation instrument 1 on the bottom surface portion of the sink 40.

Thus, the operation of moving the endoscope 20 after the preliminary cleaning from the sink 40 to the endoscope transportation instrument 1 can be performed in the sink 40. Therefore, the liquid droplets adhered to the endoscope 20 do not adhere to the floor and other members during the moving operation.

When the endoscope 20 is transported into the processing tank 31 of the endoscope cleaning/disinfecting apparatus 30, the endoscope transportation instrument 1 which holds the endoscope 20 is carried with the hook 4 being located at the upper position, and the endoscope 20 is placed with the endoscope transportation instrument 1 in the processing tank 31, as shown in FIG. 8.

In this procedure, when the endoscope transportation instrument 1 is carried, the liquid receiving portion 5 having a plate shape is located below the endoscope 20 and the holding portion 2 holding the endoscope 20. Therefore, even if liquid droplets drip from the endoscope 20, the liquid droplets fall into the recessed portion 5a of the liquid receiving portion 5. Then, the liquid receiving portion 5 is placed as-is in the processing tank 31. Therefore, the liquid droplets adhered to the endoscope 20 after the preliminary cleaning are all collected in the processing tank 31.

As described above, according to the present embodiment, the endoscope 20 can be transported while preventing the liquid droplets from dripping from the endoscope 20.

In addition, the endoscope transportation instrument 1 is configured to surround around the endoscope 20 with the support face portion 2a, the protection frame 7, and the liquid receiving portion 5 in the state holding the endoscope 20, thereby preventing the endoscope 20 from contacting other members during the transportation.

Furthermore, according to the present embodiment, the endoscope 20 subjected to the cleaning processing and disinfecting processing by the endoscope cleaning/disinfecting apparatus 30 can be suspended without being detached from the endoscope transportation instrument 1, as shown in FIG. 9, and the endoscope 20 can be subjected to drying processing and then stored.

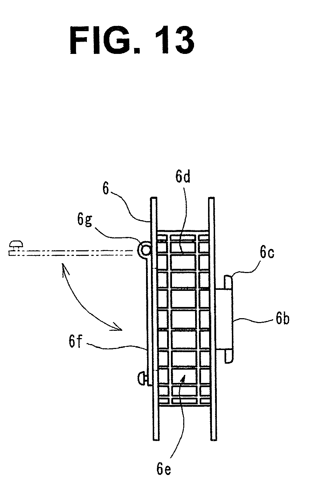

Note that, in the above-described embodiment, the winding portion 6 is a member around which the insertion portion 21 is wound. However, as shown in FIG. 13, for example, as a modified example, the winding portion 6 may be configured to be able to house therein accessories such as buttons of the endoscope 20. In the modified example shown in FIG. 13, the winding portion 6 has an outer circumferential surface 6d configured by a grating member, and has inside thereof a space 6e configured to be able to house the accessories.

The outer circumferential surface 6d forms grating, which allows the space 6e to communicate with the space around the winding portion 6. In addition, the winding portion 6 is provided with a lid member 6f for allowing access to the inside of the space 6e. The accessories can be housed in the space 6e by opening the lid member 6f. According to such a modified example, cleaning processing and disinfecting processing can be performed simultaneously on the endoscope 20 and the accessories in the processing tank 31.

Note that, in the present embodiment, the lid member 6f is configured to pivot around a hinge portion 6g to be switched between the open state and closed state. In this configuration, the hinge portion 6g is preferably located at a position close to the hook 4 in the state where the winding portion 6 is fixed to the support face portion 2a. That is, when the endoscope transportation instrument 1 is in the posture such that the hook 4 is positioned on the upper side, the hinge portion 6g is preferably located at the upper position of the lid member 6f. The hinge portion 6g is thus disposed so as to be located at the upper position of the lid member 6f when carrying the endoscope transportation instrument 1, to thereby prevent the lid member 6g from being unexpectedly brought into the open state.

### (Second Embodiment)

Hereinafter, description will be made on the second embodiment of the present invention. Hereinafter, the same constituent elements as those in the first embodiment are attached with the same reference signs and description thereof will be appropriately omitted.

An endoscope transportation instrument 10 according to the present embodiment is different from the first embodiment mainly in the configuration of a winding portion 16.

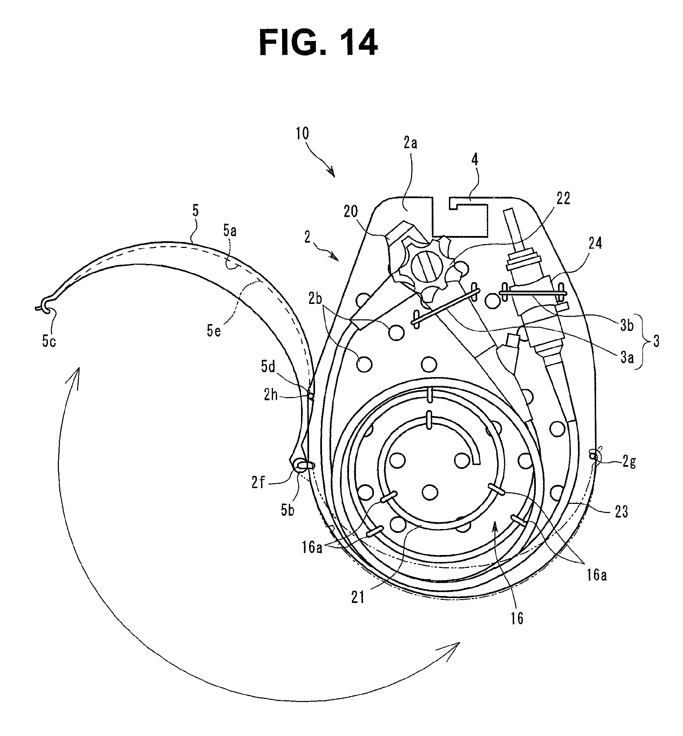

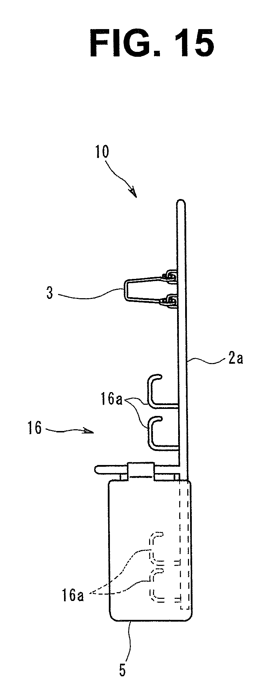

The winding portion 16 of the endoscope transportation instrument 10 according to the present embodiment is configured by a plurality of claw-like or ring-shaped inserting portions 16a fixed to the support face portion 2a, as shown in FIGS. 14 and 15. The insertion portion 21 can be latched to or inserted through the inserting portions 16a.

For example, as shown in FIG. 14, the plurality of inserting portions 16a are arranged so as to be able to support the insertion portion 21 with respect to the support face portion 2a, with the insertion portion 21 wound in a substantially spiral shape, by the insertion portion 21 being sequentially inserted through the plurality of inserting portions 16a.

Even if the winding portion 16 is configured to be fixed to the support face portion 2a as disclosed in the present embodiment, when the hook 4 is located at the upper position, the holding portion 2 and the endoscope 20 held by the holding portion 2 are located above the recessed portion 5a of the liquid receiving portion 5 similarly as in the first embodiment. Therefore, the endoscope transportation instrument 10 according to the present embodiment is also capable of transporting the endoscope 20 while preventing the liquid droplets from falling from the endoscope 20 similarly as in the first embodiment.

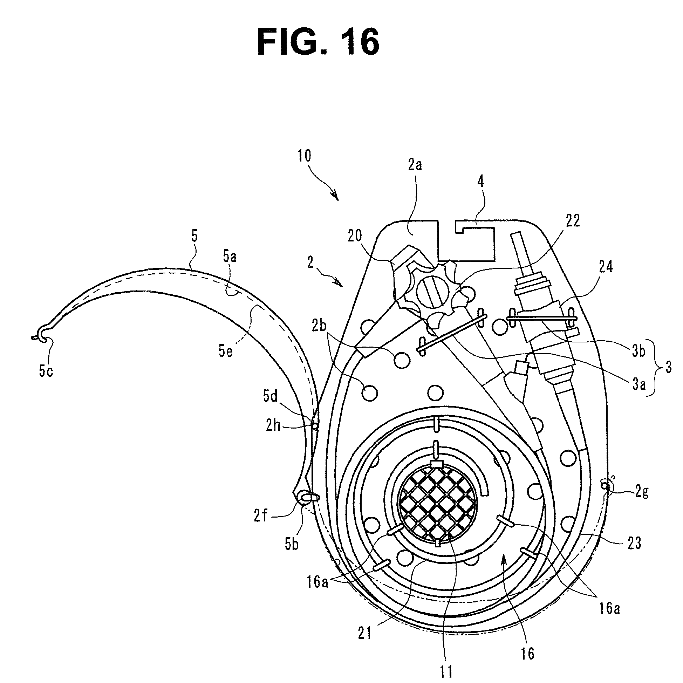

Note that the endoscope transportation instrument 10 may be provided with a basket member 11 which can house the accessories such as buttons of the endoscope 20, as shown in FIG. 16. The peripheral portion of the basket member 11 is made of a grating member, and the internal space of the basket member communicates with the external space. According to such a modified example, cleaning processing and disinfecting processing can be performed in the processing tank 31 simultaneously on the endoscope 20 and also the accessories.

Note that the present invention is not limited to the above-described embodiments, but can be modified as needed within a range not departing from the gist or thought of invention which can be read from claims and the overall specification, and also the endoscope transportation instrument modified as such is also included in the technical range of the present invention.

This application claims the benefit of Japanese Application No. 2013-148642 filed in Japan on July 17, 2013, and the above described disclosure is incorporated by reference in the present description, claims, and drawings.

## Claims

1. An endoscope transportation instrument which is an endoscope transportation instrument for transporting an endoscope in a wet state, the endoscope transportation instrument including:
a holding portion that holds the endoscope and includes a through hole for discharging liquid dripping from the endoscope when the endoscope is arranged at an upper position; and
a liquid receiving portion arranged so as to intersect with the holding portion, the liquid receiving portion including a liquid-tight recessed portion that opens upward and receives the liquid dripping from the endoscope when the endoscope held by the holding portion is arranged at the upper position.

2. The endoscope transportation instrument according to claim 1, wherein
the holding portion includes a support face portion which is a planar member on which the through hole is formed in plurality,
an endoscope latching portion that supports at least an operation portion of the endoscope with respect to the support face portion, and
a winding portion that supports an insertion portion of the endoscope with respect to the support face portion, with the insertion portion kept in a wound state.

3. The endoscope transportation instrument according to claim 1 or 2, wherein the holding portion is provided with a hook for suspending the endoscope transportation instrument such that the recessed portion opens upward.

4. The endoscope transportation instrument according to claim 3, wherein the liquid receiving portion has one end and another end, the other end being connected to the holding portion such that the one end is able to rotationally move, and in a case where the endoscope transportation instrument is suspended by the hook, the liquid receiving portion is movable between a first position at which the recessed portion opens at a position below the endoscope supported by the holding portion and a second position at which the recessed portion is away from the position below the endoscope supported by the holding portion.

5. The endoscope transportation instrument according to any one of claims 2 to 4, wherein the recessed portion of the liquid receiving portion has a bottom surface portion whose inner surface is recessed in an arc shape.

6. The endoscope transportation instrument according to any one of claims 2 to 5, wherein the winding portion is made of a cylindrical member around which the insertion portion is wound, and includes an attaching/detaching portion which is attachable to and detachable from the support face portion.
